# EUROPÄISCHE PATENTSCHRIFT

(11) **EP 3 672 536 B1**
(45) Veröffentlichungstag und Bekanntmachung des Hinweises auf die Patenterteilung: **29.09.2021**
(21) Anmeldenummer: 18739532.2
(22) Anmeldetag: 10.07.2018
(51) Int. Cl.: A61F 2/54, A61F 2/64, A61F 2/66

(54) **PROTHESENSYSTEM UND VERFAHREN ZUR ÜBERPRÜFUNG DER FUNKTIONSFÄHIGKEIT EINES PROTHESENSYSTEMS**
PROSTHETIC SYSTEM AND PROCEDURE FOR VERFYING A FUNCTIONING OF A PROSTHETIC SYSTEM
SYSTEME DE PROTHESE ET PROCEDURE POUR LA VERIFICATION DU FONCTIONNEMENT D'UN SYSTEME DE PROTHESE

(30) Priorität: 25.08.2017 DE 102017119490
(43) Veröffentlichungstag der Anmeldung: 01.07.2020
(73) Patentinhaber: Otto Bock Healthcare Products GmbH, 1110 Wien (AT)
(72) Erfinder: AMSÜSS, Sebastian, 1170 Wien (AT); GMEINER, Benjamin, 1180 Wien (AT); SCHOBESBERGER, Matthias, 2540 Bad Vöslau (AT)
(74) Vertreter: Gramm, Lins & Partner Patent- und Rechtsanwälte PartGmbB
(86) Internationale Anmeldenummer: PCT/EP2018/068624
(87) Internationale Veröffentlichungsnummer: WO 2019/037933

(56) Entgegenhaltungen:
- WO-A1-2013/088142
- WO-A2-2007/110585
- US-A- 5 888 213
- US-A1- 2002 143 405

## Beschreibung

Die Erfindung betrifft ein Prothesensystem mit zumindest zwei Sensoren, zumindest einer Steuerungseinrichtung, die mit den Sensoren gekoppelt ist und Sensorsignale der Sensoren verarbeitet, zumindest einem Aktuator, der mit der Steuerungseinrichtung gekoppelt ist und der auf der Grundlage von Steuerungssignalen der Steuerungseinrichtung aktivierbar oder deaktivierbar ist, und zumindest einer beweglich gelagerten Prothesenkomponente, die über den Aktuator verlagerbar ist. Die Erfindung betrifft ebenfalls ein Verfahren zur Überprüfung der Funktionsfähigkeit eines solchen Prothesensystems.

Eine Prothese und damit auch ein erfindungsgemäßes Prothesensystem dient als Ersatz einer nicht vorhandenen Gliedmaße. Neben dem Nachbilden eines äußeren Erscheinungsbildes dienen Prothesen oder Prothesensysteme zur Bereitstellung von Funktionen, um nach Möglichkeit die Funktion der nicht vorhandenen, natürlichen Gliedmaße vollständig oder zumindest teilweise zu ersetzen. Bei Prothesen oder Prothesensystemen sind Einrichtungen zum Festlegen der Protheseneinrichtung an einem Stumpf, beispielsweise an einem Oberschenkel- oder einem Unterarmstumpf vorhanden, die an weiteren Prothesenkomponenten angeordnet sind. Die Einrichtung zur Festlegung an einem Stumpf ist als Prothesenschaft ausgebildet, der eine formstabile Wand aufweist, die beispielsweise eine trichterförmige oder spangenartige Ausgestaltung ausbildet, um einen Stumpf darin aufzunehmen.

Zwischen dem Stumpf und dem Prothesenschaft kann ein Liner angeordnet sein, um eine Polsterung zwischen der Schaftwand und dem Stumpf bereitzustellen. Neben einem Schutz des Stumpfes dient der Liner zudem zur Sicherstellung eines Vakuums bei einem vakuumbasierten Prothesenschaftsystem oder aber zur Aufnahme der mechanischen Verriegelungseinrichtung innerhalb des Stumpfes durch ein Pinlock-System oder ein Gurtsystem. Der Liner umschließt den Stumpf vollumfänglich. Neben einer Ausgestaltung als röhrenförmiger und trichterförmiger Liner mit einer geschlossenen, distalen Endkappe, kann ein distal offener Liner ausgebildet sein, der an dem proximalen Rand des Prothesenschaftes eine Abdichtung und eine Ausrichtung bewirkt und einen direkten Kontakt zwischen einer Schaftinnenwand und einer Stumpfoberfläche gewährleistet. Der Liner kann mit Durchleitelektroden oder einer elektrisch leitenden Materialausstattung versehen sein, um myoelektrische Signale von der Hautoberfläche durch den Liner hindurch zu einem Prothesenschaft oder daran angeordnete Sensoren zu leiten.

An einem Prothesenschaft sind weitere prothetische Komponenten angeordnet, beispielsweise Gelenke, Dämpfereinrichtungen, Antriebe und Zwischenstücke, beispielsweise Prothesenkniegelenke oder Unterschenkelrohre sowie ein Prothesenfuß mit einem Prothesenknöchelgelenk. Prothesen an oberen Extremitäten sind korrespondierend aufgebaut und werden über einen Prothesenschaft an einem Oberarmstumpf oder Unterarmstumpf festgelegt. An dem distalen Ende eines Unterarmschaftes oder eines Unterarmrohres kann ein Greifelement oder eine Prothesenhand angeordnet sein, die über zumindest einen Aktuator angetrieben wird. Der Aktuator wird von einer Steuerungseinrichtung aktiviert oder deaktiviert, wobei die Steuerungseinrichtung mit Sensoren gekoppelt ist, die beispielsweise auf Grundlage myoelektrischer Signale arbeitet und individuell angepasste Aktionen auf Grundlage der Sensorsignale ausführen lässt.

Um eine solche aktive, also mit motorischen Antrieben versehene Prothese nutzen zu können, werden Sensoren, beispielsweise in Form von Elektroden, auf der Hautoberfläche des Stumpfes angeordnet oder in der Gliedmaße implantiert. Im Rahmen eines aufwendigen Lernprogrammes werden dann individuelle myoelektrische Signale den jeweiligen auszuführenden Bewegungen, die durch die Aktuatoren bewirkt werden, zugeordnet.

Nach der Montage des Prothesensystems, das aus Einzelkomponenten wie Sensoren, Steuerungseinrichtung, gegebenenfalls Verstärker sowie Aktuator, Innenschaft, Außenschaft, Energiespeicher sowie Endeffektor zusammengesetzt ist, muss das fertig montierte, auf den jeweiligen Nutzer individuell angepasste Prothesensystem auf Funktionsfähigkeit getestet werden. Dazu ist ein Kalibrierungsprozess notwendig, bei dem die Sensoren oder Elektroden positioniert und die Steuerungseinrichtung eingerichtet werden, so dass die aufgenommenen Sensorsignale eine entsprechende Aktion zur Folge haben. Dies bedeutet, dass zum Testen der Funktionsfähigkeit der Prothese der Nutzer anwesend sein muss. Aufgrund des Umstandes, dass sich auch ein Prothesennutzer zunächst an eine neue Prothese oder an ein neues Prothesensystem gewöhnen muss, ist es schwierig, die vollständige Funktionsfähigkeit eines fertig montierten Prothesensystems zu überprüfen.

Aus der WO 2013/088142 A1 ist eine Prothese einer unteren Extremität mit einer Steuereinrichtung, einer Mehrzahl von Sensoren sowie zwei Kniemotoren und einem Knöchelmotor bekannt, die in Abhängigkeit von Sensorsignalen der Sensoren von der Steuereinrichtung angesprochen werden.

Aus der WO 2007/110585 A1 ist eine Prothese einer unteren Extremität bekannt, die beispielswiese einen Schrittmotor aufweist, der in Abhängigkeit von Sensorsignalen durch eine Steuereinrichtung angesteuert wird, um Dämpfungseigenschaften eines dem Prothesenkniegelenk der Prothese zugeordneten Dämpfers zu verändern.

Aufgabe der vorliegenden Erfindung ist es, ein Prothesensystem sowie ein Verfahren bereitzustellen, mit dem die Funktionsfähigkeit eines Prothesensystems einfach und zuverlässig überprüft werden kann.

Erfindungsgemäß wird diese Aufgabe durch ein Prothesensystem mit den Merkmalen des Hauptanspruches und ein Verfahren mit den Merkmalen des nebengeordneten Anspruches gelöst. Vorteilhafte Ausgestaltungen und andere Ausführungsformen der Erfindung sind in den Unteransprüchen, der Beschreibung sowie den Figuren offenbart.

Das erfindungsgemäße Prothesensystem mit zumindest zwei Sensoren, mit zumindest einer Steuerungseinrichtung, die mit den Sensoren gekoppelt ist und Sensorsignale der Sensoren verarbeitet, mit zumindest einem Sensor, der mit der Steuerungseinrichtung gekoppelt ist und der auf der Grundlage von Steuerungssignalen der Steuerungseinrichtung aktivierbar oder deaktivierbar ist und zumindest einer beweglichen Prothesenkomponente, die über den Aktuator verlagerbar ist, sieht vor, dass in der Steuerungseinrichtung ein Standardprogramm hinterlegt oder von ihr abrufbar ist, das jedem Sensor eine Aktuatoraktion unabhängig von der Dauer und/oder Intensität des Sensorsignals zuordnet. Angetriebene Prothesensysteme bestehen aus mehreren Komponenten, unter anderem Sensoren, einer Steuerungseinrichtung sowie Aktuatoren zum Bewegen einer beweglich gelagerten Prothesenkomponente und gegebenenfalls einem Energiespeicher, um die Aktuatoren ortsunabhängig mit Energie zu versorgen, damit über die Aktuatoren, in der Regel Elektromotoren, die Prothesenkomponenten verlagert werden können. Eine Verlagerung kann insbesondere durch eine Verschwenkung um eine Schwenkachse erfolgen, wobei die Prothesenkomponente schwenkbar an einem Grundkörper gelagert ist. Alternativ zu einer Verschwenkung kann auch eine Verschiebung von dem Aktuator durchgeführt werden. Ebenfalls ist eine Kombination aus Verschiebung und Verschwenkung möglich. Solche angetriebenen Prothesensysteme basieren auf der Auswertung von Sensorsignalen. Sensoren können als implantierte Elektroden oder Oberflächenelektroden ausgebildet sein, über die Erregungspotentiale oder Aktivierungsimpulse aufgenommen werden. Solche beispielsweise myoelektrischen Signale werden von den Sensoren erfasst und mit einem hinterlegten Signalmuster verglichen. Sofern eine Übereinstimmung oder eine hinreichend genaue Übereinstimmung zwischen dem erfassten Signal oder Signalmuster und einem hinterlegten Signal oder Signalmuster vorliegt, wird eine entsprechende Aktion ausgeführt. Bei einem Prothesensystem einer oberen Extremität, beispielsweise einer Prothesenhand mit einem Prothesenschaft zur Festlegung an einem Unterarmstumpf, kann die Betätigung in einem Öffnen oder Schließen einer Prothesenhand oder einer Greifeinrichtung, beispielsweise einem sogenannten Hook liegen. Die Anpassung eines solchen Prothesensystems an den jeweiligen Nutzer ist aufwendig und umfasst neben einer mechanischen Anpassung durch eine möglichst große Übereinstimmung der Form des Prothesenschaftes an den Armstumpf auch eine steuerungstechnische Anpassung, bei der die von den Sensoren aufgenommenen Signalmuster spezifischen Aktionen eines Aktuators zugeordnet werden. Durch Anspannen und Entspannen von verschiedenen Muskeln oder Muskelgruppen kann eine Abfolge von Bewegungen der angetriebenen Prothesenkomponente erreicht werden. Dazu wird dem Nutzer des Prothesensystems vorgegeben, eine Muskelkontraktion auszuführen, die einer bestimmten Bewegung entspricht, die mit einer natürlichen Gliedmaße ausgeführt werden würde.

Sobald mit einer ausreichenden Trennschärfe ein entsprechendes Kontraktionsmuster erzeugt werden kann, wird dieses Muster einer entsprechenden Aktion der Prothesenkomponente zugeordnet.

Wird in der Steuerungseinrichtung des Prothesensystems ein Standardprogramm hinterlegt oder ist dieses Standardprogramm von der Steuerungseinrichtung abrufbar, besteht die Möglichkeit, die Funktionsfähigkeit des gesamten Prothesensystems unmittelbar nach der Montage zu testen. Unter "Abrufen" wird sowohl das Initiieren einer Datenübertragung oder das Abholen von Daten von einem externen Datenspeicher als auch die Übermittlung von Daten ohne eine Aufforderung durch die Steuerungseinrichtung verstanden. Ebenfalls ist es möglich, bei Störungen, die nach der Anpassung des Prothesensystems an den jeweiligen Nutzer auftreten, zu überprüfen, wo der Fehler zu suchen oder zu finden ist. Dazu ist in dem Standardprogramm hinterlegt, dass jedem Sensor ein Aktuatoraktion unabhängig von der Dauer und/oder Intensität des Sensorsignals zugeordnet ist. Nachdem das Prothesensystem von einem Orthopädietechniker zusammengebaut worden ist oder Funktionsprobleme einer bereits von einem Patienten genutzten Prothese aufgetreten sind, kann mit dem erfindungsgemäßen Prothesensystem schnell getestet werden, ob alle Komponenten des Prothesensystems funktionstüchtig sind oder an welcher Stelle eine Störung vorliegt. Dieses Standardprogramm wird auch als Standardklassifikator bezeichnet, der auf Signale einzelner Sensoren oder Elektroden mit vorab definierten Aktionen oder Bewegungen der jeweiligen Prothesenkomponente reagiert. Zur Überprüfung der Funktionsfähigkeit eines Prothesensystems muss ein Orthopädietechniker das Prothesensystem oder die Prothese einschalten und dann den jeweiligen Sensor aktivieren, beispielsweise mit einem Finger berühren, wobei unabhängig von der Dauer des Sensorsignals oder der Intensität des Sensorsignals eine Standardverrichtung oder eine Standardaktion durch mindestens einen Aktuator ausgeführt und die Prothesenkomponente entsprechend verlagert wird. Wird die vorgesehene Aktuatoraktion ausgeführt, lässt sich daraus schließen, dass die Energieversorgung funktioniert, der Aktuator funktioniert, sämtliche Verbindungen vorhanden sind und dass der betreffende Sensor oder die betreffende Elektrode funktionsfähig ist. Wird die vorgesehene Aktuatoraktion nicht ausgeführt, liegt ein diesbezüglicher Defekt vor, der weiter eingegrenzt werden kann. Aufgrund dieser Tatsache, dass mehrere Sensoren in einem Prothesensystem vorhanden sind, insbesondere Elektroden, wiederum insbesondere Oberflächenelektroden, die auf der Innenseite eines Prothesenschaftes oder eines Prothesenschaftsystems angeordnet sind, lassen sich unterschiedliche Aktuatoraktionen prüfen. Bei einem Prothesensystem einer oberen Extremität sind Aktuatoraktionen beispielsweise "Öffnen der Hand", "Schließen der Hand", "Drehen der Hand in die eine Richtung" oder "Drehen der Hand in die andere Richtung". Die Aktivierung eines jeweiligen Sensors oder einer jeweiligen Elektrode lässt sich sehr einfach und gezielt durch das Überstreichen mit einem Finger simulieren.

Eine andere Ausführungsform der Erfindung sieht vor, dass die Anzahl der Sensoren der Anzahl der zu verlagernden Prothesenkomponenten entspricht, so dass jede Prothesenkomponente durch Aktivieren oder Deaktivieren der jeweiligen Sensoren überprüft werden kann. Jede Funktion des Prothesensystems, die mit der Verlagerung einer Prothesenkomponente zu tun hat, kann so geprüft werden. Für das Verschwenken einer Prothesenkomponente um ein einachsiges Gelenk sind nur zwei Sensoren notwendig, die eine Verlagerung in die eine und entgegengesetzte Richtung bewirken. Für komplexere Systeme, beispielsweise eine Prothesenhand, ist eine größere Anzahl an Sensoren notwendig. Sind mehr Sensoren oder Elektroden als bewegliche Prothesenkomponenten vorhanden, beispielsweise um aufgrund einer höheren Sensoranzahl eine genauere Unterscheidung der Signalmuster erreichen zu können, können einzelne Sensorsignale einer Prothesenkomponente doppelt zugeordnet werden oder aber es findet eine Kombination mehrerer Bewegungen statt, beispielsweise "Hand drehen" und "Hand öffnen", wodurch auch eine Kombination von Steuerungssignalen und Befehlen getestet wird.

Es kann weiterhin vorgesehen sein, dass das Standardprogramm alle Funktionen der Prothesenkomponenten anspricht, um zu gewährleisten, dass das Prothesensystem vollständig hinsichtlich seiner Funktionsfähigkeit überprüft werden kann. Werden also nach dem Zusammenbau des Prothesensystems und dem Einschalten alle Sensoren nacheinander aktiviert, beispielsweise indem berührungsempfindliche Elektroden nacheinander überstrichen werden, führt das Prothesensystem alle Bewegungen nacheinander aus, so dass die untersuchende Person weiß, dass alle Elektroden oder Sensoren in der richtigen Reihenfolge angeschlossen sind. Weiterhin erfährt die untersuchende Person, dass alle Sensoren oder Elektroden einen guten Kontakt haben, dass die Steuerungseinrichtung korrekt funktioniert und dass grundsätzlich eine Mustererkennung stattfindet. Weiterhin ist nachgewiesen, dass ein Energiespeicher oder mehrere Energiespeicher korrekt angeschlossen ist oder sind, dass die Prothesenkomponenten korrekt montiert sind und dass Gelenke beweglich oder verschieblich sind und angesteuert werden. Darüber hinaus kann erkannt werden, dass keine falschen Kabelverbindungen, keine Kabeldefekte, keine Steckerdefekte oder Kontakteffekte vorliegen, so dass innerhalb kurzer Zeit für das komplette Prothesensystem vom Sensorinput bis zum Bewegungsoutput eine Komplettüberprüfung stattfinden kann, ohne dass eine komplizierte Auswärtssoftware angeschlossen oder abgespeichert werden müsste. Wird der Test bestanden, befindet sich das Prothesensystem technisch in einem einwandfreien Zustand, so dass ohne eine vorherige Individualisierung auf den Anwender eine Funktionsfähigkeitsüberprüfung stattfinden kann.

Die Sensoren sind bevorzugt berührungsempfindlich ausgebildet, so dass auf diese Art und Weise eine einfache und schnelle Überprüfung des Prothesensystems stattfinden kann. Die Steuerungseinrichtung ist frei programmierbar, so dass nach der Überprüfung und unabhängig von der Möglichkeit einer Überprüfung mit einem Standardprogramm eine Mustererkennungssoftware für die Erkennung von Signalmustern auf Grundlage der Sensordaten individuell für jeden Nutzer des Prothesensystems programmiert und adaptiert werden kann.

Jedem Sensor kann genau eine Aktuatoraktion zugeordnet sein, so dass eindeutig feststellbar ist, ob alle Sensoren funktionsfähig sind. Die Aktuatoraktion kann auch eine kombinierte Aktion aus mehreren Bewegungen mehrerer Prothesenkomponenten sein. Zur leichteren Unterscheidbarkeit und Überprüfbarkeit, ob das Prothesensystem funktionsfähig ist, ist jedem Sensor eine individuelle Aktuatoraktion zugeordnet, um zu verhindern, dass eine Fehlfunktion eines Sensors oder einer Elektrode unbemerkt bleibt.

Die Steuerungseinrichtung kann eine Schnittstelle, insbesondere eine drahtlose Schnittstelle zu einer Bedieneinrichtung wie ein Mobiltelefon, einem Tabletcomputer, einem Computer, sogenannten "wearable devices" wie Smartwatches oder Smartglases oder einer ähnlichen Einrichtung aufweisen. Alternativ oder ergänzend kann die Steuerungseinrichtung eine Schnittstelle, insbesondere eine drahtlose Schnittstelle zu einer Anzeigeeinrichtung aufweisen, um die Überprüfungsfunktion zu erweitern. Die Schnittstelle ermöglicht einen Datentransport zum Prothesensystem und von dem Prothesensystem zu einer Bedieneinrichtung und/oder einer Anzeigeeinrichtung, wodurch eine verbesserte Überprüfung der Funktionsweise erfolgen kann. Findet beispielsweise kein Verbindungsaufbau zwischen der Bedieneinrichtung oder der Anzeigeeinrichtung und der Steuerungseinrichtung statt, obwohl das Prothesensystem eingeschaltet ist, ist die Steuerungseinrichtung defekt oder die Steuereinrichtung ist nicht korrekt mit dem Energiespeicher verbunden. Sofern eine Verbindung aufgebaut werden konnte und sich das Prothesensystem in einem Überprüfungsmodus befindet, bei dem das Standardprogramm aktiviert wurde, kann eine Reaktivität der Sensoreinrichtung oder Elektroden überprüft und angezeigt werden. Über die Bedieneinrichtung, die über eine Funkverbindung mit der Prothese gekoppelt sein kann, können außerdem Steuersignale als Vorgabe für die Aktuatoren erzeugt und
an die Steuerungseinrichtung übermittelt werden. Erfolgt keine Rückmeldung beispielsweise nach dem Einschalten der Prothese oder dem Berühren der Sensoren oder des Sensors, wird auf der Bedieneinrichtung, z.B. einem Tablet oder einem Mobiltelefon eine mangelnde elektrische Verbindung angezeigt. Daraus lässt sich schließen, dass entweder der Sensor defekt ist oder sich eine Steckverbindung oder dergleichen gelöst hat. Eine korrespondierende Fehlermeldung wird dann auf der Bedieneinrichtung oder einer separaten Anzeigeeinrichtung ausgegeben. Ähnlich können weitere Überprüfungen durchgeführt und einfach dargestellt werden, wie zum Beispiel die Aktivität der Sensoren oder Elektroden bei einem Überstreichen mit einem Finger. Bleibt trotz Überstreichen und festgestellter Sensoren- oder Elektrodenaktivität eine Aktuatoraktion oder eine Bewegung der Prothese aus, muss der Fehler den Sensoren nachgeordnet lokalisiert sein, beispielsweise ein gelöster Steckkontakt oder der Motor oder die Prothesenmechanik ist defekt. Über die Bedieneinrichtung kann direkt ein Bewegungsbefehl an die Steuerungseinrichtung geschickt werden. Reagiert die Prothese darauf nicht, liegt der Fehler an der Prothese oder der Verkabelung, bewegt sich die Prothesenkomponente, liegt der Fehler bei den Elektroden oder Sensoren.

Eine Ausführungsform des Prothesensystems sieht vor, dass eine Softwareapplikation in der Bedieneinrichtung installiert ist, die dazu ausgebildet ist, auf einem Prozessor der Bedieneinheit zu laufen, wobei die Softwareapplikation ausgebildet ist, Funktionen des Prothesensystems anzuzeigen, Sensorsignale visuell zu überprüfen oder anzuzeigen, Aktuatoraktionen durch an die Steuerungseinrichtung gesendete Signale auszulösen, Fehlermeldungen, Warnmeldungen und/oder Hinweismeldungen auszugeben und/oder eine Vorgehensanleitung zur Durchführung einer Überprüfung der Funktionsfähigkeit des Prothesensystems auszugeben. Die Softwareapplikation ermöglicht beispielsweise die Anzeige von Systemfunktionen, beispielsweise durch optische und akustische Anzeigeeinrichtungen. Häufig kann eine solche Softwareapplikation auf einem Tablet oder einem Smartphone installiert sein und dort auf einem entsprechenden Prozessor laufen. Bei einer visuellen Anzeige von Systemfunktionen kann eine visuelle Überprüfung der Sensorsignale erfolgen, beispielsweise durch Darstellung des Signalweges oder durch eine optische Darstellung darüber, dass Steuersignale oder simulierte Sensorsignale, die von der Bedieneinrichtung an die Steuerungseinrichtung übermittelt wurden, nachverfolgt werden. Insbesondere bei einer Ausgestaltung der Bedieneinrichtung mit einem Touchscreen ist es möglich, Aktuatoraktionen oder Bewegungen der Aktuatoren durch an die Steuerungseinrichtung gesendete Signale, also Steuersignale oder simulierte Sensorsignale, die als Steuersignale erkannt werden, auszulösen. Die Auslösung kann manuell erfolgen, beispielsweise auf einer Bedienoberfläche, auf der das Prothesensystem und/oder die einzelnen Sensoren oder Elektroden dargestellt sind. Über die Softwareapplikation kann in der Bedieneinrichtung auch eine Rückmeldung hinsichtlich eventuell vorhandener Fehler, Gefahren oder allgemeiner Hinweise erfolgen. Dadurch ist es möglich, dem Patienten oder dem Orthopädietechniker Rückmeldungen zu geben, ob Fehler vorhanden sind, ob Fehler oder Gefährdungen zu erwarten sind und ob weitere Aspekte bei der Sicherheits-überprüfung oder bei der Nutzung und Wartung des Prothesensystems zu beachten sind.

Die Softwareapplikation kann eine Anleitung zur Durchführung der einzelnen Schritte zur Überprüfung der Funktionsfähigkeit des Prothesensystems ausgeben, ähnlich einer Bedienungsanleitung mit einem Entscheidungsbaum, über den in Abhängigkeit von den jeweiligen Rückmeldungen der Komponenten des Prothesensystems unterschiedliche weitere Schritte vorgeschlagen werden können und durchgeführt werden müssen.

Die Steuerungseinrichtung ist in einer Ausführungsform der Erfindung dergestalt ausgebildet, dass nach einer Aktivierung des Standardprogramms und dem Auslösen eines Sensorsignals eine Aktuatoraktion veranlasst und deren Ausführung oder Nichtausführung erfasst und/oder das Vorhandensein oder Nichtvorhandensein eines Sensorsignals auf einer Ausgabeeinrichtung ausgegeben wird. Die Steuerungseinrichtung ist mit den Aktuatoren oder Sensoren oder Ruckkopplungseinrichtungen verbunden, über die erfasst und an die Steuerungseinrichtung übermittelt wird, ob eine Aktion ausgeführt wurde. Weiterhin wird überprüft und über eine Rückkopplung erfasst, ob ein Steuerungssignal oder Sensorsignal vorhanden und ausgegeben worden ist. Zur Überprüfung z.B. durch einen Techniker wird eine Ausgabeeinrichtung angesteuert und die Rückmeldung ausgegeben.

Das Verfahren zur Überprüfung der Funktionsfähigkeit eines Prothesensystems mit mehreren Sensoren, zumindest einer Steuerungseinrichtung, die mit den Sensoren gekoppelt ist und Sensorsignale der Sensoren verarbeitet, mit zumindest einem Aktuator, der mit der Steuerungseinrichtung gekoppelt ist und der auf der Grundlage von Steuerungssignalen der Steuerungseinrichtung aktivierbar oder deaktivierbar ist, wobei zumindest eine beweglich gelagerte Prothesenkomponente über den Aktuator verlagerbar ist, sieht vor, dass in der Steuerungseinrichtung ein Standardprogramm hinterlegt oder von ihr abrufbar ist, mit dem jedem Sensor eine Aktuatoraktion, bevorzugt unabhängig von der Dauer und/oder Intensität des Sensorsignals, zugeordnet wird, wobei nach der Aktivierung des Standardprogrammes und dem Auslösen eines Sensorsignals eine Aktuatoraktion ausgelöst und deren Ausführung oder Nichtausführung erfasst wird und/oder das Vorhandensein oder Nichtvorhandensein eines Sensorsignals auf einer Ausgabeeinrichtung ausgegeben wird. Wird nach dem Auslösen eines Steuersignals die dem jeweiligen Steuersignal zugeordnete Aktuatoraktion ausgeführt, kann auf eine erfolgreiche Montage geschlossen werden. Wird die Aktuatoraktion nicht ausgeführt, lässt sich hieraus auf einen Defekt des Prothesensystems schließen, der aufgrund der spezifischen Zuordnung eines Sensorsignals zu einer Aktuatoraktion eingrenzbar ist. Die Aktivierung kann über eine Berührung des Sensors oder der Sensoren oder über eine mit der Prothese gekoppelte Bedieneinrichtung erfolgen, wobei die Ausgabe oder Anzeige über das Vorhandensein oder Nichtvorhandensein eines Sensorsignals oder einer Rückkopplung von der Prothese zu der Bedieneinrichtung auf der Ausgabeeinrichtung erfolgt. Die Ausgabeeinrichtung ist bevorzugt mit der Bedieneinrichtung gekoppelt, insbesondere ein Display auf einem Tablet oder einem Mobiltelefon. Die Ausgabeeinrichtung kann alternativ oder zusätzlich akustisch oder auf andere Art und Weise die Information übermitteln, ob ein Sensorsignal ausgelöst wurde, ob eine Aktivität der Sensoren feststellbar ist und/oder eine Aktion ausgeführt wurde. Dazu kann durch die Bedieneinrichtung per Fernbedienung ein Sensor mit einem elektrischen Impuls angeregt werden. Die Reaktion des Sensors, also die Erzeugung und Weiterleitung eines Sensorsignals zu der Steuerungseinrichtung kann erfasst und ausgewertet werden.

Eine andere Ausführungsform der Erfindung sieht vor, dass bei Auslösen eines Steuersignals eine Prothesenkomponente maximal in einer Verlagerungsrichtung verlagert wird. Wird nach der Auslösung des Steuersignals tatsächlich eine Verlagerung der dem Steuersignal zugeordneten Prothesenkomponente bewirkt, lässt sich aufgrund des Umstandes, dass eine vollständige Bewegung ausgeführt wird, also beispielsweise eine Maximalverdrehung in die eine Richtung oder in die anderen Richtung, der vollständige mechanische Bewegungsumfang überprüfen. Sollte die entsprechende Bewegung nicht oder nur teilweise ausgeführt werden, müssten die mechanischen Komponenten des Prothesensystems überprüft werden.

Eine andere Ausführungsform des Verfahrens sieht vor, dass vor der Ausführung einer Aktuatoraktion überprüft wird, ob die in dem Standardprogramm hinterlegte Anzahl an Sensoren mit der Steuerungseinrichtung tatsächlich gekoppelt ist. Diese Überprüfung kann unmittelbar nach dem Einschalten des Prothesensystems erfolgen. Nach dem Einschalten des Prothesensystems kann das Prothesensystem mit einer externen Anzeigeeinrichtung und/oder Bedieneinrichtung gekoppelt werden, insbesondere über eine drahtgebundene oder drahtlose Schnittstelle. Sobald eine Verbindung aufgebaut werden konnte und sich das Prothesensystem im Überprüfungsmodus befindet, kann eine Anzeige der Sensorenaktivität oder Elektrodenaktivität erfolgen, um zu überprüfen, ob alle Elektroden oder Sensoren mit der Steuerungseinrichtung, gegebenenfalls über einen Verstärker, gekoppelt sind.

Die Sensorsignale können durch einen zwischengeschalteten Verstärker oder eine Verstärkerschaltung verstärkt werden, so dass sie einfacher in der Steuereinrichtung auswertbar sind.

Für jede ausgeführte oder nicht ausgeführte Aktuatoraktion kann eine Rückmeldung an eine Bedieneinrichtung und/oder an eine Anzeigeeinrichtung gesendet werden, die mit der Steuereinrichtung über eine insbesondere drahtlose Schnittstelle gekoppelt ist.

Das Standardprogramm kann über einen Schalter oder ein Signal einer externen Bedieneinrichtung aktiviert werden, bevor ein Sensorsignal erzeugt wird. Somit wird das Prothesensystem in einen Überprüfungsmodus gebracht, in dem das Prothesensystem auf Funktionsfähigkeit geprüft wird, bevor oder nachdem eine Individualisierung durch eine Anpassung der Mustererkennungssoftware erfolgt ist.

Das Sensorsignal kann durch Aktivierung eines Sensors, z.B. durch Berühren, Annähern eines Körperteils oder andere Manipulation durch eine überprüfende Person, z.B. einem Orthopädietechniker ausgelöst werden. Damit wird überprüft, ob der Sensor oder die Elektrode als solche korrekt funktioniert, also die Komponente in einem technisch einwandfreien Zustand befindlich ist. Dazu wird die Elektrode oder der Sensor so eingesetzt und behandelt, wie es bei einer herkömmlichen Benutzung der Fall ist. Ebenso ist es möglich, dass ein Signal zur Aktivierung eines Antriebes direkt von einer Bedieneinrichtung an die Steuerungseinrichtung übermittelt wird, damit eine vollständige Überprüfung der Prothese stattfinden kann, auch wenn Sensoren nicht funktionieren oder Signale von Sensoren nicht weitergeleitet werden können.

Bevorzugt wird in dem Überprüfungsmodus die Aktuatoraktion unabhängig von der Dauer und/oder Intensität des Sensorsignals dem jeweiligen Sensor zugeordnet, so dass ein Orthopädietechniker bei einer bereits individualisierten Prothesensteuerung nicht genau die Signalmuster des jeweiligen Patienten erzeugen muss, um eine Überprüfung durchführen zu können. In dem Standardprogramm reicht es aus, ein beliebiges Signal zu erzeugen, wodurch die Überprüfung der Prothese erleichtert wird.

Da immer häufiger mehrkanalige Steuerungen eingesetzt werden, über die eine größere Anzahl an Steuerbefehlen erzeugt werden können, beispielsweise werden zur Steuerung einer Prothesenhand bis zu acht Oberflächenelektrodenpaare eingesetzt, ist in einer anderen Ausführungsform vorgesehen, dass mehrere Sensoren gleichzeitig oder in einem vorgegebenen Zeitraum nacheinander aktiviert werden und eine Aktuatoraktion veranlasst oder ausgegeben wird, die von der oder den Aktuatoraktionen abweicht, die bei einer Einzelaktivierung veranlasst oder ausgegeben werden. So kann z.B. durch ein Überstreichen von zwei Elektrodenpaaren eine andere Funktion ausgelöst werden als durch das separate, zeitlich beabstandete Überstreichen oder Aktivieren der Sensoren oder Elektrodenpaare, so dass bei komplexen Prothesen mit einer Vielzahl an Funktionen jede dieser Funktionen überprüft werden kann, auch wenn weniger Sensoren oder Elektrodenpaare als Funktionen vorhanden sind. So können beispielsweise mit drei Elektrodenpaaren bei gleichzeitiger Betätigung oder Aktivierung von Elektrodenpaaren oder Einzelaktiverungen von Elektroden bis zu sechs Funktionen einer Prothese überprüft werden.

Eine andere Ausführungsform des Verfahrens sieht vor, dass auf einem Prozessor eine Softwareapplikation ausgeführt wird, die dazu ausgestaltet ist, ein Steuerungssignal zu generieren oder ein Sensorsignal zu simulieren, das an die Steuerungseinrichtung gesendet wird. Dadurch ist es möglich, Aktuatorbewegungen oder Aktuatoraktionen manuell auszulösen, indem Signale, beispielsweise durch Berühren einer Schaltfläche auf einem Touchscreen, an die Steuerungseinrichtung gesendet werden. Durch das manuelle Erzeugen eines Steuerungssignals oder eines simulierten Sensorsignals ist es möglich, jede Funktion und jeden Sensor individuell zu überprüfen. Jedes Steuerungssignal kann individuell und einzeln an oder in der Bedieneinrichtung oder an einer anderen Eingabestelle an dem Prothesensystem erzeugt und an die Steuerungseinrichtung gesendet werden.

Die Softwareapplikation kann dazu ausgestaltet sein, Systemfunktionen auf einem Display anzuzeigen oder über eine andere Ausgabeeinrichtung, beispielsweise über eine taktile Ausgabeeinrichtung oder eine akustische Ausgabeeinrichtung, auszugeben. Die Softwareapplikation kann auch dazu ausgestaltet sein, Steuerungssignale oder simulierte Sensorsignale auf einem Display anzuzeigen, um eine Rückkopplung darüber zu erhalten, welche Steuerungssignale oder simulierten Sensorsignale erzeugt wurden und wohin diese gesendet wurden und welche Aktuatoraktionen daraufhin ausgeführt wurden.

Die Softwareapplikation kann ebenfalls dazu ausgestaltet sein, Fehlermeldungen, Warnmeldungen und/oder Hinweismeldungen über eine Ausgabeeinrichtung auszugeben, beispielsweise über ein Display oder einen Lautsprecher, der in der Bedieneinheit oder als Teil des Prothesensystems ausgebildet ist.

Mit dem erfindungsgemäßen Prothesensystem sowie mit dem erfindungsgemäßen Verfahren ist eine einfache Überprüfung des sensorischen, elektrischen, elektronischen und mechanischen Systems sowohl vor als auch nach der Anwendung an dem Patienten und Individualisierung möglich. Ein Orthopädietechniker kann somit bereits vor dem aufwendigen Anpassen der Steuerung an den Patient überprüfen, ob die Prothese grundsätzlich funktionsfähig ist, ob alle Stecker oder Verbindungen intakt sind oder ob und wo Störungen auftreten. Diese Störungen können lokalisiert werden, so dass eine Reparatur oder Korrektur vereinfacht wird. Ebenso wird die Fehlersuche bei einer bereits individualisierten Prothese erleichtert. Es kann auch eine Fernwartung oder Ferndiagnose über die Bedieneinrichtung durchgeführt werden, ohne dass der Orthopädietechniker vor Ort sein muss, was den Zeitaufwand für eine Reparatur verringert. Es kann überprüft werden, ob alle Komponenten vorhanden sind, ohne dass eine Anpassung an einen Patienten erfolgt sein muss. Insbesondere werden Sensoren, Aktuatoren und Kabel, Verbindungen sowie gegebenenfalls Energiespeicher und die mechanischen Komponenten der Protheseneinrichtung überprüft. Nach dem Einschalten können die jeweiligen Komponenten eine Rückmeldung geben oder aktiv melden, ob sie angeschlossen sind.

Nachfolgend wird die Erfindung anhand der beigefügten Figuren näher erläutert. Es zeigen:
Figur 1 - eine schematische Darstellung eines Prothesensystems; sowie
Figur 2 - ein Ablaufdiagramm einer Überprüfung.

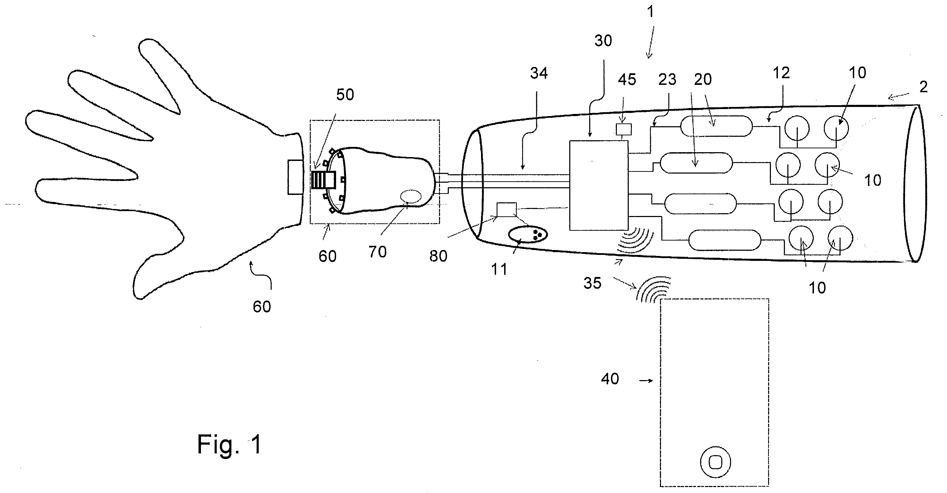

Figur 1 zeigt in einer schematischen Darstellung eine beispielhafte Darstellung eines Prothesensystems 1 in der Gestalt eines Prothesenhandsystems mit einem Prothesenschaft 2, über den das Prothesensystem an einem Unterarmstumpf festlegbar ist. Alternativ zu einer Ausgestaltung als Prothese einer oberen Gliedmaße ist es möglich und vorgesehen, das Prothesensystem 1 als Prothese einer unteren Gliedmaße auszugestalten. Ebenso ist es möglich, den Prothesenschaft 2 zur Aufnahme eines Oberarmstumpfes auszugestalten. Innerhalb des Prothesenschaftes 2 sind eine Vielzahl von Sensoren 10 in der Gestalt von Oberflächenelektroden angeordnet, im dargestellten Ausführungsbeispiel sind vier Elektrodenpaare als Sensoren 10 auf der Innenoberfläche des Prothesenschaftes 2 angeordnet. Über die Sensoren können myoelektrische Signale bei Muskelkontraktionen aufgenommen werden. Die Sensoren 10 erfassen Oberflächenpotentiale und können auch berührungsempfindlich sein. Weiterhin ist in dem Prothesenschaft 2 oder an dem Prothesenschaft 2 für jedes Elektrodenpaar ein Verstärker 20 vorgesehen, der mit dem Elektrodenpaar über eine Leitung 12 verbunden ist. Von dem Verstärker 20 wird über eine Leitung 23 eine Verbindung zu einer Steuerungseinrichtung 30 hergestellt, so dass Sensorsignale von den Sensoren 10 über die Leitung 12, den Verstärker 20 und die Leitung 23 mit der Steuerungseinrichtung 30 gesendet werden. Ebenfalls mit der Steuerungseinrichtung 30 verbunden ist ein Schalter 45 zur Aktivierung eines Standardprogrammes, das in der Steuerungseinrichtung 30 abgelegt ist.

In der Steuerungseinrichtung 30 ist eine Schnittstelle 35 zur Verbindung der Steuerungseinrichtung 30 und damit des Prothesensystems 1 mit einer externen Bedieneinrichtung 40, beispielsweise in Gestalt eines Mobiltelefons, eines Tablets oder eines Computers ausgebildet. Statt einer drahtlosen Schnittstelle 35, wie im dargestellten Ausführungsbeispiel gezeigt, kann diese auch über eine Steckerverbindung verwirklicht werden.

Ein Ein-Aus-Schalter 11 ist an dem Prothesenschaft 2 angeordnet, um das elektrische und elektronische System des Prothesensystems 1 zu aktivieren oder zu deaktivieren. Der Schalter 11 ist mit einem Energiespeicher 80 gekoppelt, der in dem Prothesenschaft 2 angeordnet ist und über den einerseits die Steuerungseinrichtung 30 und die Verstärker 20 und andererseits ein Aktuator 70 mit elektrischer Energie versorgt werden. Der Aktuator 70 weist zumindest einen Elektromotor auf, der auf der Basis von Steuerungssignalen, die über Leitungen 34 von der Steuerungseinrichtung 30 zu dem Aktuator 70 geleitet werden, aktivierbar und deaktivierbar ist. Der Aktuator 70 ist im endmontierten Zustand Teil des Prothesensystems 1 und wird bevorzugt innerhalb des Prothesenschaftes 2 oder innerhalb einer aktuierbaren Prothesenkomponente 60 gelagert, die Teil des Prothesensystems ist. Die Prothesenkomponente 60 ist im dargestellten Ausführungsbeispiel als Prothesenhand ausgebildet, die über den Aktuator 70 antreibbar ist, kann aber auch als andere Prothesenkomponente ausgebildet sein, beispielsweise als ein sogenannter Hook, ein individuelles Greifwerkzeug, eine andere Prothese der oberen Extremität oder als Prothese der unteren Extremität, beispielsweise als Prothesenfuß, der dann nicht mehr über Elektroden 10 an dem Unterarm, sondern z.B. an dem Unterschenkel aktiviert wird. Mit der Prothesenhand 60 können verschiedene Verrichtungen ausgeführt werden, beispielsweise kann die Prothesenhand 60 relativ zu dem Prothesenschaft 2 verdreht werden, um eine Verlagerung um eine parallel zur Längserstreckung des Prothesenschaftes 2 gerichtete Drehachse zu ermöglichen. Ebenfalls kann oder können mit dem Aktuator 70 ein oder mehrere Prothesenfinger der Prothesenhand 60 bewegt werden, so dass eine Schließbewegung oder eine Öffnungsbewegung der Prothesenhand 60 bewirkt werden kann.

Nach einer Endmontage befindet sich der Aktuator 70 innerhalb eines Prothesenkonnektors 50 und der wiederum innerhalb des Prothesenschaftes 2. Die Prothesenkomponente 60 ist an dem Prothesenkonnektor 50 festgelegt. Zusätzlich ist es möglich, dass innerhalb der Prothesenkomponente 60 weitere Antriebe oder Aktuatoren, Gelenke, Kraftübertragungseinrichtungen, Getriebeeinrichtungen und dergleichen angeordnet sind. Darüber hinaus können weitere Energiespeicher 80 an oder innerhalb des Prothesenschaftes 2 befestigt und mit den elektrischen und elektronischen Komponenten gekoppelt sein. Der Prothesenschaft 2 kann mehrteilig ausgebildet sein und beispielsweise einen Innenschaft und einen Außenschaft aufweisen, um den Prothesenschaft 2 auszubilden. Der Außenschaft stellt eine mechanisch hoch belastbare, rohrartige oder sich konisch erweiternde Aufnahme für einen Stumpf einer Gliedmaße sowie Befestigungseinrichtungen für die mechanischen Komponenten und insbesondere die mindestens eine Prothesenkomponente 60 bereit. Ein Innenschaft ist aus einem vergleichsweise weicheren, elastischen Material ausgebildet und dient zur Herstellung eines unmittelbaren Kontaktes mit der Hautoberfläche des Stumpfes. Die Sensoren 10 oder Elektroden sind dann an dem Innenschaft befestigt, so dass es sich bei der Endmontage, insbesondere beim Zusammenstecken des Außenschaftes und des Innenschaftes ergeben kann, dass sich ein Kabel löst oder bricht. Ein solcher Fehler kann bei herkömmlichen Prothesensystemen, die auf einer Mustererkennung basierend arbeiten, nur durch Anlegen der Prothese an den Patienten, Starten eines Kalibrierungsprozesses und Testen aller Steuersignale in Zusammenarbeit mit dem Anwender gefunden werden. Dies bedeutet, dass der Anwender oder Patient während des Zusammenbaus oder einer Funktionsüberprüfung herkömmlicher Prothesensysteme anwesend sein muss. Insbesondere können für den Fall eines nicht geübten Anwenders Fehler des herkömmlichen Prothesensystems nicht eindeutig identifiziert werden, da Fehlsteuerungen durch ungeübte Kontraktionen bedingt sein können.

Da in der Steuerungseinrichtung 30 ein Standardklassifikator oder ein Standardprogramm abgelegt ist, das auf Aktivitäten einzelner Elektroden oder Sensoren 10 mit vorbestimmten Bewegungen der Prothesenkomponente 60 reagiert, unabhängig davon, ob andere Elektroden oder Sensoren 10 aktiviert werden und wie lange oder wie intensiv ein Sensorsignal ausgegeben wird, ist es möglich, auf einfache Art und Weise das Prothesensystem 1 vollständig auch auf Funktionsfähigkeit zu überprüfen. Das Standardprogramm in der Steuerungseinrichtung 30 reagiert auf eine Aktivität einer ersten Elektrode 10 mit der Aktion "Hand öffnen", auf die Aktivität einer zweiten Elektrode 10 mit "Hand schließen", die Aktivität einer dritten Elektrode 10 mit "Hand nach oben drehen" und die Aktivität einer vierten Elektrode 10 mit "Hand nach unten drehen". Die Aktivierung einer einzelnen Elektrode oder eines Sensors 10 lässt sich durch einfaches und gezieltes Überstreichen oder Berühren mit einem Finger simulieren, so dass bei Auslösen eines Sensorsignals durch einen Kontakt mit einem Finger oder dergleichen eine Aktuatoraktion ausgeführt wird.

Für eine einfache Funktionsüberprüfung würde ein Orthopädietechniker nach dem Zusammenbau des Prothesensystems 1 diese über den Ein-Aus-Schalter 11 einschalten und dann mit einem Finger alle Sensoren 10 oder Elektrodenpaare 10 nacheinander überstreichen. Führt das Prothesensystem mit der Prothesenkomponente 60 oder der Prothesenhand 60 die vorgesehenen Bewegungen nacheinander vollständig aus, weiß der Orthopädietechniker, dass alle Sensoren 10 oder Elektroden in der richtigen Reihenfolge angeschlossen sind, dass diese Sensoren 10 oder Elektroden einen guten Kontakt haben, dass eine Verstärkung der Signale in den Verstärkern 20 stattfindet und dass die Steuerungseinrichtung 30 mit den Sensoren 10 in Gestalt der Elektroden verbunden sind. Bei einer vollständigen Ausführung der vorgesehenen Bewegungen wird dadurch auch angezeigt, dass die Steuerungseinrichtung 30 korrekt funktioniert und grundsätzlich eine Mustererkennung durchgeführt und die Ansteuerung der Prothesenkomponente 60 in jeglicher Richtung erfolgen kann. Die Energiespeicher 80 sind angeschlossen und die mechanischen Komponenten der Prothesenkomponente 60, also Gelenke, Kraftübertragungseinrichtungen, Getriebe, Teleskopeinrichtung oder dergleichen sind korrekt angeschlossen und werden wie vorgesehen angesteuert. Nach dem Einschalten über den Schalter 11 und dem Überstreichen der Sensoren 10 wird das gesamte Prothesensystem mit Mechanik, Elektrik und Elektronik vollständig in einer sehr kurzen Zeitspanne geprüft. Um das in der Steuerungseinrichtung 30 abgelegte Standardprogramm oder den Standardklassifikator zu aktivieren, kann über den Schalter 45 das entsprechende Programm aktiviert oder deaktiviert werden. So kann nach dem Einschalten über den Ein-Aus-Schalter 11 zunächst das Standardprogramm über die Aktivierung des Auswahlschalters 45 erfolgen, bevor die Sensoren 10 berührt werden. Nach Abschluss der Überprüfung wird das Standardprogramm ausgeschaltet, so dass eine Kalibrierung des Prothesensystems 1 auf den jeweiligen Prothesennutzer erfolgen kann. Dazu ist die Steuerungseinrichtung 30 im Rahmen einer Mustererkennung frei programmierbar oder trainierbar.

Sofern sämtliche in dem Standardprogramm vorgesehenen Bewegungen der Prothesenkomponenten korrekt ausgeführt worden sind, kann ein Orthopädietechniker sicher sein, dass das Prothesensystem 1 in einem elektrisch, elektronisch und mechanisch einwandfreien Zustand befindlich ist, bevor es an den Endanwender ausgeliefert wird.

Alternativ oder zusätzlich zu einem mechanischen Schalter 45 kann der Standardkalibrator oder das Standardprogramm über die Schnittstelle 35 an die Steuerungseinrichtung übermittelt werden, bevor die Überprüfung des Prothesensystems 1 erfolgt.

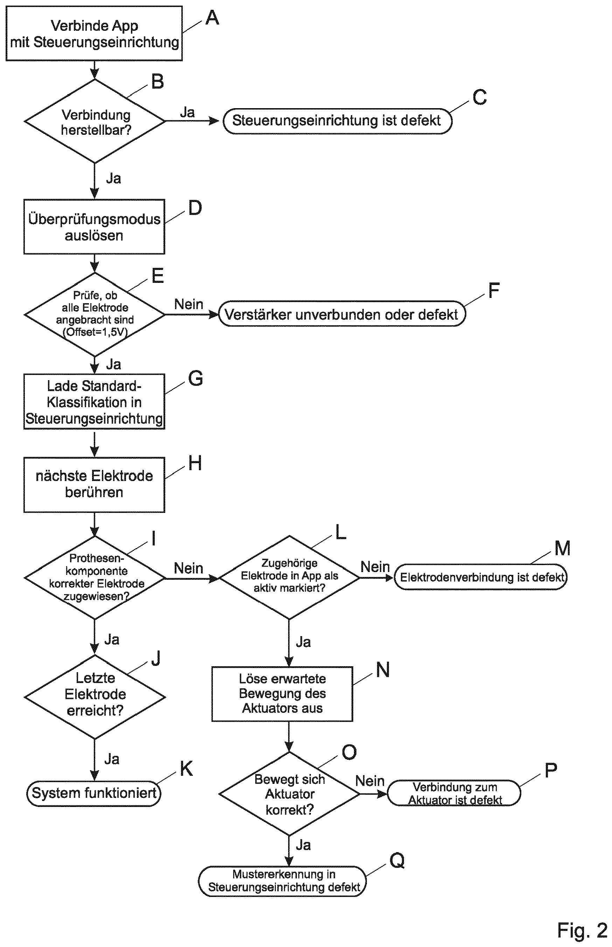

Sofern die Überprüfung nicht erfolgreich war, also mindestens eine derProthesenkomponenten 60 nicht die vorgesehenen Aktionen oder Aktuatoraktionen ausführt, kann der Orthopädietechniker eine entsprechende Korrektur oder Reparatur vornehmen. Diese Art der Überprüfung ist insbesondere bei der Neuerstellung einer Prothese vorteilhaft. Ergänzend oder alternativ kann eine erweiterte Prüfung über eine Fernbedienung und/oder bei bereits angepasster auf den Patienten eingestellter Prothese mit Steuerung durchgeführt werden, um die Fehlerquelle zu identifizieren. Der mögliche Ablauf hierzu ist in dem Ablaufdiagramm gemäß Figur 2 dargestellt.

Zunächst wird nach dem Einschalten über den Schalter 11 in einem Schritt A die Bedieneinrichtung 40 in Gestalt beispielsweise eines Mobiltelefons, Tablets, wearable devices oder Computers über die Schnittstelle 35 mit der Steuerungseinrichtung 30 gekoppelt. Die Schnittstelle 35 kann als eine drahtlose Schnittstelle oder auch eine kabelgebundene Schnittstelle ausgebildet sein. In einer Abfrage B wird überprüft, ob eine Verbindung zwischen der Bedieneinrichtung 40, die auch eine optische Anzeige beinhaltet, mit der Steuerungseinrichtung 30 hergestellt werden konnte. Sollte dies nicht der Fall sein, kann im Schritt C festgestellt werden, dass die Steuerungseinrichtung 30 defekt ist.

Konnte eine Verbindung festgestellt werden, ergibt sich im Schritt D, dass der Überprüfungsmodus eingestellt wird. Zunächst wird abgefragt, ob alle Sensoren 10 oder alle Elektroden mit der Steuerungseinrichtung 30 verbunden sind. Sollte dies nicht der Fall sein, ist die Verbindungsleitung 23 zu dem Verstärker 20 oder der Verstärker 20 selbst defekt, was im Schritt F ausgegeben wird. Sofern alle Sensoren 10 oder Elektrodenpaare detektiert wurden und verbunden sind, wird der Standardklassifikator in der Steuerungseinrichtung 30 aktiviert oder in diesen geladen. Dies geschieht im Schritt G. Anschließend werden die Sensoren 10 oder Elektroden 10, die als Hautkontaktelektroden ausgeführt sind, mit einem Finger nacheinander berührt oder über eine Software elektrisch oder elektronisch angeregt, was im Schritt H erfolgt.

Bewegen sich sämtliche Prothesenkomponenten 60 in der vorgesehenen Art und Weise und in der vorgesehenen Reihenfolge, was in dem Schritt I abgefragt wird, und ist die letzte Elektrode überprüft, was in Schritt J abgefragt wird, ist das Prothesensystem 1 korrekt montiert und funktionsfähig, was im Schritt K ausgegeben wird. Ist die letzte Elektrode oder Sensor 10 noch nicht überprüft, wurden also noch nicht alle Elektroden berührt oder angeregt, wird der Vorgang ab Schritt H wiederholt, bis die letzte Elektrode abgefragt wurde. Die Schritte G bis K können bevorzugt auch ohne die externe Bedieneinrichtung 40 ausgeführt werden. Jede einzelne Elektrode oder jeder einzelne Sensor 10 wird somit durch das Verfahren individuell überprüft. In dem Prothesensystem können mehrere Prothesenkomponenten 60 vorhanden sein beispielsweise elektrische Ellenbögen oder elektrische Kniegelenke oder Fußgelenke. Jede Prothesenkomponente 60 kann einen oder mehrere Aktuatoren 70 enthalten. In dem Prothesensystem, das eine Prothesenhand aufweist, kann eine Komponente 60 als Prothesenkonnetor 50 zusammen mit dem Aktuator 70 zur Bewegung der Prothesenhand ausgebildet sein. Der Aktuator 70 ist für die Bewegung der Prothesenhand in dem Bereich des Handgelenkes zuständig, um eine Drehung um eine Drehachse auszuüben. Es können auch mehrere Aktuatoren 70 vorhanden sein, um mehrere verschiedene Drehbewegungen ausführen zu können. Unterschiedliche Drehbewegungen können auch mit nur einem Aktuator 70 ausgeführt werden. Zusätzlich ist die Prothesenhand als solche eine Prothesenkomponente 60, in der mehrere Aktuatoren 70 angeordnet sein können, um die Prothesenfinger relativ zu einem Chassis zu bewegen. Innerhalb der Prothesenfinger können auch Aktuatoren 70 angeordnet sein, um Fingerglieder relativ zueinander und/oder relativ zu dem Chassis zubewegen. Mehrere Komponenten 60 sind zu dem Prothesensystem zusammengesetzt oder zusammensetzbar. Nebst mindestens einem Aktuator kann jede Prothesenkomponente auch über mindestens einen Konnektor 50 verfügen, welcher dazu dient, die Komponenten untereinander oder mit dem Prothesenschaft 2 zu verbinden.

Sollte in dem Überprüfungsschritt I die Prothesenkomponente 60 nicht die korrekten Bewegungen ausführen, wird im Schritt L abgefragt, ob die korrespondierende Elektrode oder der korrespondierende Sensor 10 in der Bedieneinrichtung 40 oder in der App als aktiv hervorgehoben wurde. Sofern dies nicht der Fall ist, ist die Verbindung 12 von der Elektrode 10 zu dem Verstärker 20 defekt, was in dem Schritt M angezeigt wird.

Wird die Elektrode 10 als aktiv in der Bedieneinrichtung 40 angezeigt, wird im Schritt N die gewünschte Bewegung der Prothesenkomponente 60 manuell oder in der Bedieneinrichtung 40 ausgelöst. Im Schritt O erfolgt die Abfrage, ob die Prothesenkomponente 60 die gewünschte Bewegung oder die gewünschten Bewegungen vollständig und in der richtigen Reihenfolge ausführt. Sollte dies nicht der Fall sein, ist die Verbindung 34 von der Steuerungseinrichtung 30 zu dem Aktuator 70 defekt, was in Schritt P ausgegeben wird. Sollte die Prothesenkomponente 60 die jeweils vorgegebene Bewegung korrekt ausführen, kann im Schritt Q identifiziert werden, dass die Mustererkennungssoftware innerhalb der Steuerungseinrichtung 30 defekt ist.

Zeigt die Bedieneinrichtung 40 keine Verbindungsmöglichkeit zu dem Prothesensystem 1 an, obwohl diese über den Schalter 11 eingeschaltet ist, sind entweder die Stecker von der Steuerungseinrichtung 30 zu dem Aktuator 70 gelöst oder die Steuerungseinrichtung 30 ist defekt.

Sollten bereits Patientenkalibrierungsdaten in der Steuerungseinrichtung 30 oder in dem Prothesensystem 1 gespeichert sein, so kann über die Bedieneinrichtung 40 das Standardprogramm oder der Standardklassifikator aktiviert oder in das Prothesensystem 1 übertragen werden.

Werden im Schritt H die Sensoren 10 mit einem Finger überstrichen oder anderweitig aktiviert, wird dieses Überstreichen mit dem Finger oder Aktivieren beispielsweise per Icon auf der Bedieneinrichtung 40 dargestellt. Signalisiert ein Icon beim Überstreichen oder Aktivieren der entsprechenden Elektrode keine Aktivität, so kann der Orthopädietechniker auf einen Fehler dieser Elektrode oder dieses Sensors 10 schließen. Wenn alle Elektroden beim Überstreichen oder Aktivieren korrekt als aktiv angezeigt werden, aber die entsprechende Prothesenbewegung ausbleibt, liegt der Fehler darin, dass eine Kabelverbindung defekt oder die Prothesenkomponente 60 defekt ist. Wird über die Bedieneinrichtung 40 oder die Überprüfungssoftware direkt ein Bewegungsbefehl geschickt, beispielsweise "Hand öffnen" und reagiert die Prothesenhand 60 nicht, liegt der Fehler nicht in der Software oder in der Elektronik, sondern in einer defekten Kabelverbindung, in einer defekten Aktuatoreinheit 70 oder in der Prothesenkomponente 60. Bewegt sich die Prothesenkomponente 60 aufgrund des Befehls über die separate Bedieneinrichtung 40, jedoch nicht beim Überstreichen oder Aktivieren der eigentlich dafür vorgesehenen Sensoren 10, sind die Sensoren 10 defekt.

Durch das Durchlaufen des Prüfplans gemäß Figur 2 kann sehr schnell die korrekte Funktionsweise und Funktionsfähigkeit des Prothesensystems sichergestellt werden oder aber, sofern ein Fehler auftritt, die Fehlerquelle schnell identifiziert und einfach beseitigt werden, so dass nach dem Zerlegen des Prothesensystems eine schnelle Fehlerbehebung stattfinden kann.

## Patentansprüche

1. Prothesensystem mit zumindest zwei Sensoren (10),
mit zumindest einer Steuerungseinrichtung (30), die mit den Sensoren (10) gekoppelt ist und Sensorsignale der Sensoren (10) verarbeitet,
mit zumindest einem Aktuator (70), der mit der Steuerungseinrichtung (30) gekoppelt ist und der auf der Grundlage von Steuerungssignalen der Steuerungseinrichtung (30) aktivierbar oder deaktivierbar ist, und
mit zumindest einer beweglich gelagerten Prothesenkomponente (60), die über den Aktuator (70) verlagerbar ist, **dadurch gekennzeichnet, dass** in der Steuerungseinrichtung (30) ein Standardprogramm hinterlegt oder von ihr abrufbar ist, das jedem Sensor (10) eine Aktuatoraktion unabhängig von der Dauer und/oder Intensität des Sensorsignals zuordnet.

2. Prothesensystem nach Anspruch 1, **dadurch gekennzeichnet, dass** das Standardprogramm alle Funktionen der Prothesenkomponenten (60) anspricht.

3. Prothesensystem nach einem der voranstehenden Ansprüche, **dadurch gekennzeichnet, dass** ein Energiespeicher (80) in dem Prothesensystem (1) integriert oder diesem zugeordnet ist, der mit dem zumindest einen Aktuator (70) gekoppelt ist.

4. Prothesensystem nach einem der voranstehenden Ansprüche, **dadurch gekennzeichnet, dass** die Sensoren (10) berührungsempfindlich ausgebildet sind.

5. Prothesensystem nach einem der voranstehenden Ansprüche, **dadurch gekennzeichnet, dass** die Steuerungseinrichtung (30) frei programmierbar und das Standardprogramm verschieden zu einem Anwendungsprogramm ist.

6. Prothesensystem nach einem der voranstehenden Ansprüche, **dadurch gekennzeichnet, dass** jedem Sensor (10) genau eine Aktuatoraktion zugeordnet ist.

7. Prothesensystem nach einem der voranstehenden Ansprüche, **dadurch gekennzeichnet, dass** jedem Sensor (10) eine individuelle Aktuatoraktion zugeordnet ist.

8. Prothesensystem nach einem der voranstehenden Ansprüche, **dadurch gekennzeichnet, dass** die Steuerungseinrichtung (30) eine Schnittstelle (35) zu einer Bedieneinrichtung (40) und/oder Anzeigeeinrichtung aufweist.

9. Prothesensystem nach Anspruch 8, **dadurch gekennzeichnet, dass** eine Softwareapplikation in der Bedieneinrichtung (40) installiert ist, die dazu ausgebildet ist, auf einem Prozessor der Bedieneinheit (40) zu laufen, wobei die Softwareapplikation ausgebildet ist, Funktionen des Prothesensystems anzuzeigen, Sensorsignale visuell zu überprüfen und/oder anzuzeigen, Aktuatoraktionen durch an die Steuerungseinrichtung (30) gesendete Signale auszulösen, Fehler-, Warn- und/oder Hinweismeldungen auszugeben und/oder eine Vorgehensanleitung zur Durchführung einer Überprüfung der Funktionsfähigkeit des Prothesensystems auszugeben.

10. Prothesensystem nach einem der voranstehenden Ansprüche, **dadurch gekennzeichnet, dass** die Steuerungseinrichtung (30) nach einer Aktivierung des Standardprogramms und dem Auslösen mindestens eines Sensorsignals mindestens eine Aktuatoraktion veranlasst und deren Ausführung oder Nichtausführung erfasst und/oder das Vorhandensein oder Nichtvorhandensein eines Sensorsignals auf einer Ausgabeeinrichtung ausgibt.

11. Verfahren zur Überprüfung der Funktionsfähigkeit eines Prothesensystems (1) mit mehreren Sensoren (10), zumindest einer Steuerungseinrichtung (30), die mit den Sensoren (10) gekoppelt ist und Sensorsignale der Sensoren (10) verarbeitet, zumindest einem Aktuator (70), der mit der Steuerungseinrichtung (30) gekoppelt ist und der auf der Grundlage von Steuerungssignalen der Steuerungseinrichtung (30) aktivierbar oder deaktivierbar ist, wobei zumindest eine beweglich gelagerte Prothesenkomponente (60) über den Aktuator (70) verlagerbar ist, **dadurch gekennzeichnet, dass** in der Steuerungseinrichtung (30) ein Standardprogramm hinterlegt oder von ihr abrufbar ist, mit dem jedem Sensor (10) eine Aktuatoraktion zugeordnet wird und nach einer Aktivierung des Standardprogramms und dem Auslösen eines Sensorsignals eine Aktuatoraktion veranlasst und deren Ausführung oder Nichtausführung erfasst wird und/oder das Vorhandensein oder Nichtvorhandensein eines Sensorsignals auf einer Ausgabeeinrichtung ausgegeben wird.

12. Verfahren nach Anspruch 11, **dadurch gekennzeichnet, dass** bei Auslösen eines Sensorsignals eine Prothesenkomponente (60) maximal in einer Verlagerungsrichtung verlagert wird.

13. Verfahren nach Anspruch 11 oder 12, **dadurch gekennzeichnet, dass** vor Ausführung einer Aktuatoraktion überprüft wird, ob die in dem Standardprogramm hinterlegte Anzahl an Sensoren (10) mit der Steuerungseinrichtung (30) gekoppelt ist.

14. Verfahren nach einem der Ansprüche 11 bis 13, **dadurch gekennzeichnet, dass** die Sensorsignale verstärkt werden.

15. Verfahren nach einem der Ansprüche 11 bis 14, **dadurch gekennzeichnet, dass** für jede ausgeführte oder nicht ausgeführte Aktuatoraktion eine Rückmeldung an eine Bedieneinrichtung (40) und/oder Anzeigeeinrichtung gesendet wird.

16. Verfahren nach einem der Ansprüche 11 bis 15, **dadurch gekennzeichnet, dass** das Standardprogramm über einen Schalter (45) oder ein Signal einer externen Bedieneinrichtung (40) aktiviert wird, bevor ein Sensorsignal abgenommen oder gemessen wird.

17. Verfahren nach einem der Ansprüche 11 bis 16, **dadurch gekennzeichnet, dass** das Sensorsignal durch Aktivierung eines Sensors oder durch eine Bedieneinrichtung (40) an die Steuerungseinrichtung (30) übermittelt wird.

18. Verfahren nach einem der Ansprüche 11 bis 17, **dadurch gekennzeichnet, dass** die Aktuatoraktion unabhängig von der Dauer und/oder Intensität des Sensorsignals dem jeweiligen Sensor (10) zugeordnet wird.

19. Verfahren nach einem der Ansprüche 11 bis 18, **dadurch gekennzeichnet, dass** mehrere Sensoren (10) gleichzeitig oder in einem vorgegebenen Zeitraum nacheinander aktiviert werden und eine Aktuatoraktion veranlasst oder ausgegeben wird, die von der oder den Aktuatoraktionen abweicht, die bei einer Einzelaktivierung veranlasst oder ausgegeben werden.

20. Verfahren nach einem der Ansprüche 11 bis 19, **dadurch gekennzeichnet, dass** auf einem Prozessor eine Softwareapplikation ausgeführt wird, die dazu ausgestaltet ist, ein Steuerungssignal zu generieren oder ein Sensorsignal zu simulieren, das an die Steuerungseinrichtung (30) gesendet wird.

21. Verfahren nach Anspruch 20, **dadurch gekennzeichnet, dass** die Softwareapplikation dazu ausgestaltet ist, Systemfunktionen auf einem Display anzuzeigen oder über eine andere Ausgabeeinrichtung auszugeben.

22. Verfahren nach Anspruch 20 oder 21, **dadurch gekennzeichnet, dass** die Softwareapplikation dazu ausgestaltet ist, Steuerungssignale oder simulierte Sensorsignale auf einem Display anzuzeigen.

23. Verfahren nach einem der Ansprüche 20 bis 22, **dadurch gekennzeichnet, dass** die Softwareapplikation dazu ausgestaltet ist, Fehler-, Warn- und/oder Hinweismeldungen über eine Ausgabeeinrichtung auszugeben.

24. Verfahren nach einem der Ansprüche 20 bis 23, **dadurch gekennzeichnet, dass** die Softwareapplikation dazu ausgestaltet ist, über eine Ausgabeeinrichtung verschiedene Verfahrensschritte vorzugeben und durch das Überprüfungsverfahren zu führen.

## Claims

1. A prosthesis system having at least two sensors (10),
having at least one control device (30), which is coupled to the sensors (10) and processes sensor signals of the sensors (10),
having at least one actuator (70), which is coupled to the control device (30) and can be activated or deactivated on the basis of control signals of the control device (30), and
having at least one movably mounted prosthesis component (60), which can be displaced by means of the actuator (70), **characterized in that** a standard program, which assigns an actuator action to each sensor (10) independently of the duration and/or intensity of the sensor signal, is stored in the control device (30) or can be called up thereby.

2. The prosthesis system as claimed in claim 1, **characterized in that** the standard program operates all functions of the prosthesis components (60).

3. The prosthesis system as claimed in one of the preceding claims, **characterized in that** an energy store (80), which is coupled to the at least one actuator (70), is integrated in the prosthesis system (1) or assigned thereto.

4. The prosthesis system as claimed in one of the preceding claims, **characterized in that** the sensors (10) are configured to be touch-sensitive.

5. The prosthesis system as claimed in one of the preceding claims, **characterized in that** the control device (30) is freely programmable, and the standard program is different to an application program.

6. The prosthesis system as claimed in one of the preceding claims, **characterized in that** precisely one actuator action is assigned to each sensor (10).

7. The prosthesis system as claimed in one of the preceding claims, **characterized in that** an individual actuator action is assigned to each sensor (10).

8. The prosthesis system as claimed in one of the preceding claims, **characterized in that** the control device (30) comprises an interface (35) to an operating device (40) and/or a display device.

9. The prosthesis system as claimed in claim 8, **characterized in that** a software application, which is configured to run on a processor of the operating device (40), is installed in the operating device (40), the software application being configured to display functions of the prosthesis system, to visually check and/or display sensor signals, to trigger actuator actions by signals sent to the control device (30), to output error, warning and/or advice messages, and/or to output procedural instructions for carrying out a check of the functionality of the prosthesis system.

10. The prosthesis system as claimed in one of the preceding claims, **characterized in that** after activation of the standard program and triggering of at least one sensor signal, the control device (30) induces at least one actuator action and records the execution or non-execution thereof and/or outputs the presence or absence of a sensor signal on an output device.

11. A method for checking the functionality of a prosthesis system (1) having a plurality of sensors (10), at least one control device (30), which is coupled to the sensors (10) and processes sensor signals of the sensors (10), at least one actuator (70), which is coupled to the control device (30) and can be activated or deactivated on the basis of control signals of the control device (30), at least one movably mounted prosthesis component (60) being displaceable by means of the actuator (70), **characterized in that** a standard program, by which an actuator action is assigned to each sensor (10) and, after activation of the standard program and triggering of a sensor signal, an actuator action is induced and the execution or non-execution thereof is recorded and/or the presence or absence of a sensor signal is output on an output device, is stored in the control device (30) or can be called up thereby.

12. The method as claimed in claim 11, **characterized in that**, when a sensor signal is triggered, a prosthesis component (60) is displaced maximally in a displacement direction.

13. The method as claimed in claim 11 or 12, **characterized in that**, before the execution of an actuator action, a check is made as to whether the number of sensors (10) stored in the standard program are coupled to the control device (30).

14. The method as claimed in one of claims 11 to 13, **characterized in that** the sensor signals are amplified.

15. The method as claimed in one of claims 11 to 14, **characterized in that** for each executed or not executed actuator action, a report is sent to an operating device (40) and/or display device.

16. The method as claimed in one of claims 11 to 15, **characterized in that** the standard program is activated by means of a switch (45) or a signal of an external operating device (40), before a sensor signal is received or measured.

17. The method as claimed in one of claims 11 to 16, **characterized in that** the sensor signal is sent to the control device (30) by activation of a sensor or by an operating device (40).

18. The method as claimed in one of claims 11 to 17, **characterized in that** the actuator action is assigned to the respective sensor (10) independently of the duration and/or intensity of the sensor signal.

19. The method as claimed in one of claims 11 to 18, **characterized in that** a plurality of sensors (10) are activated simultaneously or successively in a predetermined period of time, and an actuator action is induced or output which differs from the actuator action which is induced or output in the event of a single activation.

20. The method as claimed in one of claims 11 to 19, **characterized in that** a software application which is configured to generate a control signal or to simulate a sensor signal, which is sent to the control device (30), is run on a processor.

21. The method as claimed in claim 20, **characterized in that** the software application is configured to display system functions on a display or to output them by means of another output device.

22. The method as claimed in claim 20 or 21, **characterized in that** the software application is configured to display control signals or simulated sensor signals on a display.

23. The method as claimed in one of claims 20 to 22, **characterized in that** the software application is configured to output error, warning and/or advice messages via an output device.

24. The method as claimed in one of claims 20 to 23, **characterized in that** the software application is configured to specify various method steps via an output device and to carry out the checking method.

## Revendications

1. Système de prothèse comportant
au moins deux capteurs (10),
au moins un dispositif de commande (30) qui est couplé aux capteurs (10) et qui traite les signaux des capteurs (10),
au moins un actionneur (70) qui est couplé au dispositif de commande (30) et qui peut être activé ou désactivé sur la base de signaux de commande du dispositif de commande (30), et
au moins un composant de prothèse (60) monté de manière mobile, qui peut être déplacé par l'intermédiaire de l'actionneur (70),
**caractérisé en ce qu'**un programme standard est mémorisé dans le dispositif de commande (30) ou peut être appelé à partir de celui-ci, lequel programme attribue à chaque capteur (10) une action d'actionneur indépendamment de la durée et/ou de l'intensité du signal du capteur.

2. Système de prothèse selon la revendication 1,
**caractérisé en ce que** le programme standard met en œuvre toutes les fonctions des composants de prothèse (60).

3. Système de prothèse selon l'une des revendications précédentes, **caractérisé en ce qu'**un accumulateur d'énergie (80) est intégré dans le système de prothèse (1) ou est associé à celui-ci et est couplé audit au moins un actionneur (70).

4. Système de prothèse selon l'une des revendications précédentes, **caractérisé en ce que** les capteurs (10) sont de conception tactile.

5. Système de prothèse selon l'une des revendications précédentes, **caractérisé en ce que** le dispositif de commande (30) est librement programmable, et le programme standard est différent d'un programme d'application.

6. Système de prothèse selon l'une des revendications précédentes, **caractérisé en ce que** chaque capteur (10) est associé précisément à une action de l'actionneur.

7. Système de prothèse selon l'une des revendications précédentes, **caractérisé en ce que** chaque capteur (10) est associée à une action individuelle de l'actionneur.

8. Système de prothèse selon l'une des revendications précédentes, **caractérisé en ce que** le dispositif de commande (30) présente une interface (35) avec un dispositif de manœuvre (40) et/ou un dispositif d'affichage.

9. Système de prothèse selon la revendication 8,
**caractérisé en ce qu'**une application logicielle est installée dans le dispositif de manœuvre (40), qui est conçue pour être exécutée sur un processeur de l'unité de manœuvre (40), l'application logicielle étant conçue pour afficher des fonctions du système de prothèse, pour contrôler visuellement et/ou afficher des signaux de capteurs, pour déclencher des actions de l'actionneur au moyen des signaux envoyés au dispositif de commande (30), pour émettre des messages d'erreur, d'avertissement et/ou d'information et/ou pour émettre des instructions pour effectuer un contrôle de l'aptitude au fonctionnement du système de prothèse.

10. Système de prothèse selon l'une des revendications précédentes, **caractérisé en ce que** le dispositif de commande (30), après activation du programme standard et déclenchement d'au moins un signal de capteur, initie au moins une action de l'actionneur et détecte son exécution ou sa non-exécution et/ou émet la présence ou l'absence d'un signal de capteur sur un dispositif de sortie.

11. Procédé de contrôle de l'aptitude au fonctionnement d'un système de prothèse (1) comprenant plusieurs capteurs (10), au moins un dispositif de commande (30) qui est couplé aux capteurs (10) et qui traite les signaux des capteurs (10), au moins un actionneur (70) qui est couplé au dispositif de commande (30) et qui peut être activé ou désactivé sur la base de signaux de commande du dispositif de commande (30), au moins un composant de prothèse (60) monté de manière mobile pouvant être déplacé par l'intermédiaire de l'actionneur (70),
**caractérisé en ce qu'**un programme standard est mémorisé dans le dispositif de commande (30) ou peut être appelé à partir de celui-ci, programme avec lequel une action de l'actionneur est associée à chaque capteur (10) et, après activation du programme standard et déclenchement d'un signal de capteur, une action de l'actionneur est initiée et son exécution ou sa non-exécution est détectée et/ou la présence ou l'absence d'un signal de capteur est émise sur un dispositif de sortie.

12. Procédé selon la revendication 11,
**caractérisé en ce que**, lorsqu'un signal de capteur est déclenché, un composant de prothèse (60) est déplacé au maximum dans une direction de déplacement.

13. Procédé selon la revendication 11 ou 12,
**caractérisé en ce que**, avant l'exécution d'une action de l'actionneur, on vérifie si le nombre de capteurs (10) mémorisé dans le programme standard est couplé au dispositif de commande (30).

14. Procédé selon l'une des revendications 11 à 13,
**caractérisé en ce que** les signaux des capteurs sont amplifiés.

15. Procédé selon l'une des revendications 11 à 14,
**caractérisé en ce que** pour chaque action de l'actionneur exécutée ou non exécutée, un retour d'information est envoyé à un dispositif de commande (40) et/ou à un dispositif d'affichage.

16. Procédé selon l'une des revendications 11 à 15,
**caractérisé en ce que** le programme standard est activé par l'intermédiaire d'un commutateur (45) ou d'un signal d'un dispositif de manœuvre externe (40) avant qu'un signal de capteur soit pris ou mesuré.

17. Procédé selon l'une des revendications 11 à 16,
**caractérisé en ce que** le signal de capteur est transmis au dispositif de commande (30) par activation d'un capteur ou par un dispositif de manoeuvre (40).

18. Procédé selon l'une des revendications 11 à 17,
**caractérisé en ce que** l'action de l'actionneur est associée au capteur respectif (10) indépendamment de la durée et/ou de l'intensité du signal du capteur.

19. Procédé selon l'une des revendications 11 à 18,
**caractérisé en ce que** plusieurs capteurs (10) sont activés simultanément ou successivement dans une période de temps prédéterminée, et une action de l'actionneur est initiée ou émise qui diffère de l'action ou des actions de l'actionneur initiées ou émises lors d'une activation individuelle.

20. Procédé selon l'une des revendications 11 à 19,
**caractérisé en ce qu'**une application logicielle est exécutée sur un processeur, qui est conçue pour générer un signal de commande ou pour simuler un signal de capteur qui est envoyé au dispositif de commande (30).

21. Procédé selon la revendication 20,
**caractérisé en ce que** l'application logicielle est conçue pour afficher les fonctions du système sur un écran d'affichage ou pour les émettre via un autre dispositif de sortie.

22. Procédé selon la revendication 20 ou 21,
**caractérisé en ce que** l'application logicielle est conçue pour afficher sur un écran d'affichage des signaux de commande ou des signaux de capteur simulés.

23. Procédé selon l'une des revendications 20 à 22,
**caractérisé en ce que** l'application logicielle est conçue pour émettre des messages d'erreur, d'avertissement et/ou d'information via un dispositif de sortie.

24. Procédé selon l'une des revendications 20 à 23,
**caractérisé en ce que** l'application logicielle est conçue pour spécifier diverses étapes du procédé via un dispositif de sortie et pour mener à travers le procédé de contrôle.
